# EUROPEAN PATENT SPECIFICATION

(11) **EP 3 740 286 B1**
(45) Date of publication and mention of the grant of the patent: **07.02.2024**
(21) Application number: 19701467.3
(22) Date of filing: 14.01.2019
(51) Int. Cl.: A61K 31/665, A61P 35/04

(54) **COMPOSITIONS FOR ELIMINATING BACTERIAL PROMOTORS OF COLORECTAL CANCER BY INTRALUMINAL APPLICATION**
ZUSAMMENSETZUNGEN ZUR BESEITIGUNG BAKTERIELLER PROMOTOREN VON KOLOREKTALKARZINOM DURCH INTRALUMINALE ANWENDUNG
COMPOSITIONS POUR ÉLIMINER LES PROMOTEURS BACTÉRIENS DU CANCER COLORECTAL PAR APPLICATION INTRALUMINALE

(30) Priority: 17.01.2018 DK PA201870030; 14.06.2018 DK PA201870392
(43) Date of publication of application: 25.11.2020
(73) Proprietor: Reponex Pharmaceuticals A/S, 2970 Hørsholm (DK)
(72) Inventor: UTTENTHAL, Lars Otto, 28009 Madrid (ES); BJØRN, Torsten, 3490 Kvistgård (DK)
(74) Representative: Zacco Denmark A/S
(86) International application number: PCT/EP2019/050798
(87) International publication number: WO 2019/141622

(56) References cited:
- WO-A1-2015/038731
- US-A1- 2014 107 092
- US-A1- 2017 196 937
- YUICHI KONAKAJIMA ET AL: "Morphological and structural responsies of Fusobacterium nucleatum to fosfomycin.", SHIKA KISO IGAKKAI ZASSHI - JAPANESE JOURNAL OF ORAL BIOLOGY, vol. 27, no. 3, 1 January 1985 (1985-01-01), pages 782-793, XP055573663, JP ISSN: 0385-0137, DOI: 10.2330/joralbiosci1965.27.782

## Description

### Field of invention

The present invention provides compositions comprising fosfomycin for local application to eliminate or reduce the colonization or infection of colorectal cancer (CRC) tumors by fusobacteria and other susceptible microorganisms and the presence of bacterial biofilm associated with such tumors or their development. The purpose of the invention is to reduce the tumor growth and resistance to chemotherapy or radiotherapy that is associated with the infection, and to reduce the pro-carcinogenic action ascribed to the biofilm. It is relevant to the fields of oncology and gastroenterology as well as to other fields comprising the prevention and treatment of colorectal cancers.

### Background of invention

The term "fusobacteria" refers to members of the genus *Fusobacterium* of Gram-negative anaerobic bacteria, species of which, including *F. nucleatum,* are found in the oral cavity, where they play a role in periodontal disease. Genomic sequences of fusobacteria have been found to be enriched in CRC tumors, with a prominence of DNA sequences related to *F. nucleatum* (Kostic et al 2012). An overabundance of *F*. *nucleatum* RNA was also detected in such tumors (Castellarin et al 2012). Fusobacteria target colorectal adenocarcinoma cells by means of their Fap2 lectin, which specifically binds to the D-galactose-β(1-3)-N-acetyl-D-galactosamine (Gal-GalNAc) residues of the surface carbohydrates of colorectal and certain other adenocarcinomas. Once in the tumor, fusobacteria can enhance cellular proliferation (Rubinstein et al 2013; Chen et al 2017; Yang et al 2017), create a tumor-favorable inflammatory environment (Kostic et al 2013) and protect the cancer cells against killing by natural killer cells and tumor-infiltrating T cells. *F. nucleatum* also promotes the resistance of CRC to chemotherapy (Yu et al 2017), and, because similar cellular mechanisms are involved, to radiotherapy. High fusobacterial abundance in CRC correlates with poor disease outcome (Flanagan et al 2014). At the same time, there has been a call for antibacterial agents targeted specifically to fusobacteria to avoid the side effects of systemically administered broad-spectrum antibiotics.

Fusobacteria such as *F. nucleatum* are also present in bacterial biofilms associated with CRC tumors, particularly with tumors of the ascending colon. In these biofilms, bacteria of the *Bacteroides* and *Prevotella* genera often the most prominent, together with *Enterobacteriaceae* such as *Escherichia coli,* while *Clostridium* spp., actinobacteria and various bacilli, including the putatively protective lactobacilli, can also be found in addition to fusobacteria. The biofilm associated with CRC tumors is characteristically thick and continuous, invading the deeper mucous layer and lying in contact with colonic epithelial cells both over the tumor and in adjacent normal epithelium. Tissues underlying the biofilm show a decrease or alteration of the tumor suppressor E-cadherin, enhanced expression of the pro-inflammatory and angiogenic cytokine IL-6 and the proliferation-associated Ki-67 protein, as well as Stat3 activation. This points to a pro-carcinogenic effect of the biofilm (Dejea et al 2016). Among specific ways in which the bacteria present in the biofilm can initiate CRC is a direct mutagenic effect on DNA or interference with host DNA repair. This is the case for enterotoxigenic *Bacteroides fragilis* (ETBF), superoxide-producing *Enterococcus faecalis,* and colibactin-producing *E. coli.* Many of the biofilm bacteria can enhance Wnt-mediated signaling pathways or other specific pro-inflammatory pathways that are commonly mutated and/or overexpressed in CRC, as seen in the above-mentioned bacterial strains and *F. nucleatum.* There are also local immunological effects of the biofilm, as illustrated by the promotion of a tumorigenic Th17 response by ETBF that has been demonstrated in a mouse model (Chung et al 2018). These mechanisms promoting CRC initiation are also likely to play a similar role in CRC progression (Drewes et al 2016). Colonic biofilm and CRC tumors, especially tumors of the ascending colon, seem to enter into a symbiotic relationship, whereby each component can promote the formation and growth of the other in a vicious circle. A factor in this is an increased production of *N¹,N¹²*-diacetylspermine, which originates from both the biofilm and the tumor (Johnson et al 2015). The inventors do not want to be bound by any theories but consider that the technical effect of the local administration of fosfomycin is due to the removal of pro-carcinogenic biofilm from the colonic mucosa which will break the vicious circle, reduce CRC tumor size, help restore susceptibility to chemotherapy and radiotherapy and tend to normalize immune responses to the tumors, as well as reduce the initiation of recurrent tumors. US 2014/107092 discloses the treatment of colorectal cancer by administering an effective amount of an antibiotic effective against fusobacteria. The use of fosfomycin is however not mentioned.

### Summary of the invention

Accordingly, the present invention seeks to target fusobacterial infection or colonization of colorectal tumors and eliminate CRC-associated bacterial biofilm by providing pharmaceutical compositions for treating these conditions by the local administration of the compositions into the lumen of the bowel where the tumors and or biofilm are located, said compositions comprising essentially:
A composition comprising the antibiotic fosfomycin as an active ingredient for reducing or eliminating infection or colonization of colorectal cancer tumors by fusobacteria and the bacterial biofilm associated with such tumors, wherein the composition is administered locally into the bowel lumen.

The composition for use according to the item above, which further comprises one or more additional antimicrobial or antibiotic agents as active ingredients.

The composition for use according to the previous item, wherein the one or more additional antimicrobial or antibiotic agents is/are chosen from a non-limiting list comprising metronidazole or a carbapenem such as ertapenem, or meropenem, or imipenem.

The composition for use according to the previous items, wherein the active ingredient fosfomycin is in the form of fosfomycin trometamol or fosfomycin disodium.

The composition for use according to the previous items, wherein the active ingredients are formulated as a dry powder or granulate to be dissolved in an aqueous medium before delivery into the bowel lumen.

The composition according to the previous item, wherein the dry powder or granulate, and/or the aqueous medium for dissolution, contains one or more additional substances to promote the penetration of the active ingredients into the tumors and biofilm, said additional substances being chosen from the list of dimethyl sulfoxide, glycofurol, polyoxyethylene lauryl ether, sodium taurocholate, and sodium caprate.

In the following detailed description of the invention, details of the scope of the invention and its practical performance will be given.

### Detailed description of the invention

The purpose of the present invention is to provide a means of reducing or eliminating the colonization or infection of colorectal cancer tumors by fusobacteria as well as to eliminate the bacterial biofilm associated with these tumors. This is achieved by the direct local administration of a solution of appropriate antibiotics into the bowel lumen at a level which ensures the application of the solution to the site of the tumor and/or biofilm. The colonization of the tumors by fusobacteria is associated with increased tumor growth and resistance to both endogenous antitumor immune mechanisms and exogenous antitumor therapies. Some of the mechanisms by which these effects are achieved have been elucidated. It is therefore proposed that the elimination or drastic reduction of fusobacteria colonization of these tumors will reverse these adverse effects and increase the efficacy of other anticancer therapies directed at these tumors. Eliminating the bacterial biofilm associated with these tumors (i.e. covering the tumor and adjacent areas of normal colonic epithelium) will also exert these effects, as well as removing the pro-carcinogenic action of the biofilm that may promote tumor recurrence and tumor growth.

Local administration of the antibiotics via the bowel lumen, e.g. through application via an endoscope or catheter or, in some cases, as a retention enema, will result in a very high antibiotic concentration at the luminal surface of the tumor and the biofilm. The antibiotics will penetrate into the tumor cells where the fusobacteria are located and into the overlying and adjacent biofilm to achieve bactericidal concentrations, while at the same time being only poorly absorbed into the blood, so that any systemic side effects of the antibiotics are substantially reduced.

Quantitative polymerase chain reaction analysis of *F. nucleatum* DNA in the tumors demonstrates that the bacteria are concentrated in the superficial, luminal portion of the tumors (Yamamura et al 2017). Antibacterial treatment by applying high concentrations of antibiotic from the luminal side should be particularly effective in eliminating the bulk of the bacterial load at the same time minimizing side effects associated with conventional systemic administration of the broad spectrum antibiotic. The same consideration applies to the elimination of the overlying and adjacent bacterial biofilm.

### Active ingredients of the compositions of the invention

Compositions according to the present invention comprise essentially the antibiotic fosfomycin, either alone or in combination with one or more additional antibiotics, which may be chosen from a non-limiting list comprising metronidazole, and carbapenems such as ertapenem, meropenem and imipenem.

### Fosfomycin

Fosfomycin is the international non-proprietary name of a broad-spectrum antibiotic isolated and characterized in 1969 from *Streptomyces fradiae* strains under the name phosphomycin or phosphonomycin (Hendlin et al 1969). Its structure was determined to be (-)(IR, 2S)-1,2-epoxypropylphosphonic acid (Christensen et al 1969), with the systematic (IUPAC) name [(2R,3S)-3-methyloxiran-2-yl]phosphonic acid and a formula weight of 138.1 Da. Fosfomycin is bactericidal and inhibits bacterial cell wall biosynthesis by inactivating the enzyme UDP-N-acetylglucosamine-3-enolpyruvyltransferase, also known as MurA (Brown et al 1995). This enzyme catalyzes the committed step in peptidoglycan biosynthesis, the ligation of phosphoenolpyruvate to the 3'-hydroxyl group of UDP-N-acetylglucosamine to form N-acetylmuramic acid. Fosfomycin is a phosphoenolpyruvate analogue that inhibits MurA by alkylating an active site cysteine residue. The antibiotic enters the bacterial cell via the glycerophosphate transporter.

Given this mechanism of action, fosfomycin has a broad bactericidal spectrum, being active against aerobic genera such as *Staphylococcus, Streptococcus, Enterococcus, Neisseria, Escherichia, Proteus* (indole-negative), *Serratia, Salmonella, Shigella, Pseudomonas, Haemophilus,* and *Vibrio,* less active against indole-positive *Proteus* spp., *Klebsiella* and *Enterobacter* spp. Of particular relevance to the present invention, it is active against the anaerobic genus *Fusobacterium,* as well as the anaerobic genera *Peptostreptococcus* (including *Peptoniphilus, Finegoldia* and *Anaerococcus*)*.* However, it is inactive against the anaerobic *Bacteroides fragilis* group of bacteria.

There is a low prevalence of bacterial resistance to fosfomycin in the community, and studies of the prevalence of resistant bacteria after the introduction of fosfomycin have shown either no increase or only a modest increase in the prevalence of resistant organisms. However, prolonged exposure to the antibiotic may enable bacteria to evolve resistance by selection of mutants that lack the glycerophosphate transporter pathway. Alternative mechanisms of resistance involve the loss of the inducible hexose phosphate transporter, a Cys-Asp mutation in MurA, or acquistion of plasmids coding for the fosfomycin inactivating enzymes fosA and fosB (in addition to the chromosomal fosX in *Listeria monocytogenes*)*.* The mutant strains may, however, also show reduced pathogenicity (Karageorgopoulos et al 2012). This may explain why the emergence of bacterial resistance is seen on prolonged exposure *in vitro,* but much less frequently *in vivo.* The appearance of resistant bacterial strains in controlled clinical trials of orally or intravenously administered fosfomycin has been 3.0% overall, with a maximum of 15% for *Pseudomonas aeruginosa.* In general, fosfomycin is seen to be a valuable addition to the therapeutic armament against multidrug-resistant organisms.

Fosfomycin has been shown to have the capacity to favor phagocytosis and act as an immunomodulator. It is accumulated by polymorphonuclear leukocytes to reach concentrations that are up to twice those of the extracellular fluid, but does not affect their cellular functions, while still exerting a bactericidal effect on *Staphylococcus aureus* (Höger et al 1985).

With respect to its uptake into intestinal mucosal cells, Martinez et al (2013) have demonstrated the rapid uptake of fosfomycin into IPEC-J2 cells exposed to fosfomycin calcium at 580 µg/mL, to reach an intracellular concentration approaching 30 µg/mL within 15 minutes. Such high concentrations of fosfomycin are difficult to reach with standard systemic route of administration. Therefore, much higher concentrations of fosfomycin are required to kill fusobacteria in colorectal cancer cells. In accordance with the invention such concentrations are readily reached with locally administered fosfomycin solution.

Fosfomycin is known to penetrate readily through tissue barriers and into bacterial biofilm (Reffert & Smith 2014), so that high, bactericidal concentrations of fosfomycin will result from the direct application of fosfomycin solution to bacterial biofilm covering and adjacent to the colorectal tumors. Fosfomycin inhibits the adhesion to epithelial cells of various bacterial species, including those involved in biofilm formation (Gobernado 2003). Fosfomycin can also break up biofilms to enhance the permeability of other antibiotics,

The chief adverse effects of fosfomycin are gastric irritation from orally administered fosfomycin disodium, evidence of allergy in the form of transient rashes (0.3% of cases) and eosinophilia (0.2%), as well as transiently raised liver enzymes (0.3% of cases) from systemically administered fosfomycin (Gobernado 2003). In general, however, fosfomycin displays remarkably low toxicity, so that when high doses of fosfomycin disodium are given systemically, it is the sodium load that is the dose-limiting factor.

Fosfomycin shows a considerable synergism in bactericidal effect on a large number of strains of organisms from the susceptible genera mentioned, when used in combination with a large number of antibiotics of the penicillin, cephalosporin, aminoglycoside, macrolide and lincosamide types. While early studies showed a synergistic effect on about 70-100% of tested strains for various antibiotic combinations, subsequent more extensive studies showed synergy rates of 36-74%. The remaining strains showed merely additive effects and an inhibitory effect was only seen in one or two individual antibiotic combinations on an individual bacterial strain (Gobernado 2003). The fact that fosfomycin shows synergy with many individual antibiotics and indeed abrogates the toxicity of many other antibiotics, including the nephrotoxicity and ototoxicity of the aminoglycosides, favors the use of fosfomycin in combination with other antibiotics to produce a potent bactericidal action and compensate for any development of fosfomycin resistance during more prolonged treatment.

The principal forms of fosfomycin that come within the scope of this invention are:
i) Fosfomycin disodium, formula weight 182.0 Da, pH of 5% solution 9.0-10.5. This salt is hygroscopic, highly soluble in water and shows a high bioavailability, but is locally irritant if un-neutralized.
ii) Fosfomycin calcium monohydrate, formula weight 194.1 Da, pH of 0.4% solution 8.1-9.6. This salt is sparingly soluble in water and not hygroscopic, but is less irritating to the stomach when used for oral treatment, when its bioavailability in terms of entering the systemic circulation may be as low as 12% (Bergan 1990).
iii) Fosfomycin trometamol, formula weight 259.2 Da, pH of 5% solution 3.5-5.5. This salt is highly soluble in water and is well tolerated when given orally, when it shows a bioavailability of about 40%.

When the name "fosfomycin" is used herein, it refers to an inorganic or organic salt of fosfomycin as exemplified by the principal forms above, and the dose of fosfomycin refers to the amount of the free acid form of fosfomycin present in the salt.

The preferred form of fosfomycin for the compositions of the present invention is fosfomycin trometamol, which shows long-term stability as a readily soluble granulate when stored in sealed sachets at room temperature. However, fosfomycin disodium can also be used, requiring storage in sealed vials because of its marked hygroscopic property.

Compositions according to the present invention may comprise fosfomycin such that single doses are in the range of 400 milligram to 4 gram.

### Metronidazole

This semi-synthetic antibiotic, which is active against a number of anaerobic bacteria including bacteria of the *B*. *fragilis* group and *Fusobacterium* spp., is well known to prior art. It has been used systemically in combination with systemic fosfomycin for prophylaxis against wound infection after abdominal surgery. The intracellular penetration of metronidazole appears to be by rapid passive diffusion, so that intracellular levels approach equilibrium with extracellular levels within 15 minutes (Hand et al 1987). Compositions according to the present invention may comprise metronidazole such that single doses are in the range of 100 milligram to 1 gram.

### Carbapenems

The carbapenems are members of the beta-lactam class of antibiotics, which, like the penicillins and cephalosporins, exert a bactericidal action by inhibiting bacterial cell wall synthesis by a different mechanism than that of fosfomycin. Hence the beta-lactam antibiotics, including the carbapenems, characteristically show a synergic bactericidal action with fosfomycin on most bacterial species against which both types of antibiotic are active. However, the carbapenems exhibit a broader spectrum of activity than most penicillins and cephalosporins, being active against anaerobic bacteria such as the B. *fragilis* group, *Prevotella* spp. and *Fusobacterium* spp. They can hence be used in place of metronidazole to exert a bactericidal action on the anaerobic bacteria that are largely insensitive to fosfomycin. Regarding the intracellular penetration of the carbapenems, bactericidal intracellular concentrations of ertapenem in a mouse macrophage cell line are achieved at extracellular concentrations as low as 15 µg/mL (Tang et al 2012), and the cellular to extracellular concentration ratios of meropenem in human macrophages were always high (range 3-12) at extracellular concentrations ranging from 0.125 to 1 µg/mL (Cuffini et al 1993). Compositions according to the present invention may comprise ertapenem, or meropenem or imipenem such that single doses are in the range of 100 milligram to 1 gram.

### Medical indications

The primary indication for the use of the compositions described is the presence of one or more colorectal tumors arising from the colonic or rectal mucosa and/or the presence of bacterial biofilm covering the colonic or rectal mucosa whether or not the biofilm is associated with an adenocarcinoma, an adenoma or polyp or mucosal dysplasia. The indication will typically be diagnosed in relation to colonoscopy. Treatment with the compositions described can typically be administered through the endoscope during the same session or at a later procedure by the intraluminal instillation of a solution of the composition in the bowel section where the mucosal pathology is observed. The initial treatment can be followed up by further treatments at the discretion of the treating clinician.

An indication for treatment may also be provided detecting urinary, fecal, blood or tissue markers of pro-carcinogenic biofilm or driver organisms. Currently, such markers could be the presence of colibactin-producing *E. coli,* enterotoxigenic *B. fragilis, F. nucleatum* and *Porphyromonas gingivalis,* as well as other microbes, especially from the oral microbiota. Many markers are under investigation, but none has yet shown a diagnostic sensitivity or specificity quite high enough to be independently diagnostic of either CRC or colonic biofilm, so that they currently have a screening function to select subjects for colonoscopy. Among markers with a promising diagnostic sensitivity and specificity is urinary *N¹,N¹²*-diacetylspermine, which, as stated above, is produced in increased amounts by CRC-associated bacterial biofilm.

If it is known or suspected that a tumor has penetrated through the bowel wall or has metastasized to lymph nodes or remote tissues, the treating clinician may decide to supplement the local antibiotic treatment with systemic antibiotic treatment, as live microorganisms may be found in metastases of colorectal carcinomas.

Tumors and biofilm present in the rectum and sigmoid colon, because of their location, offer the opportunity for treatment with the compositions made up as retention enemas. In certain circumstances, larger volumes of enema containing the compositions can be used to treat regions of the colon more remote from the rectum.

The use of the compositions described may also be indicated during neoadjuvant radiotherapy or chemotherapy, e.g. with bevacizumab and/or cytotoxic drugs.

### Formulations

In a preferred embodiment, the composition comprises fosfomycin trometamol supplied as a dry granulate to be dissolved in a supplied pharmaceutically acceptable carrier, preferably an aqueous carrier or diluent. The carrier may be simply water or water containing pharmaceutically acceptable auxiliary substances or adjuvants, including, without limitation, buffering agents and/or tonicity adjusting agents to ensure a neutral pH and isotonicity of the fosfomycin solution. In another embodiment, the composition comprises fosfomycin disodium, such as fosfomycin disodium formulated for intravenous injection, supplied as freeze-dried powder in a capped glass vial. This is similarly dissolved in a pharmaceutically acceptable aqueous carrier or diluent.

In a further embodiment, the composition comprises fosfomycin trometamol and an additional antibiotic, such as metronidazole, or a carbapenem such as ertapenem, meropenem or imipenem, both antibiotics being supplied as a dry powder or granulate to be dissolved in the aqueous carrier.

The pH value of the solutions resulting from the formulations according to the present invention may have a pH of between 4 and 8, preferably between 6.5 and 7.5 and most preferably between 7 and 7.5. The antibiotic powders or granulates and the aqueous diluents supplied may individually contain buffering agents and/or tonicity adjusting agents to ensure a neutral pH and isotonicity of the combined antibiotic solution. They may also contain one or more approved biocompatible thickening agents that do not bind the active ingredients of the composition, such as hydroxypropyl methylcellulose or a large number of other biocompatible thickening agents known to the art, in order to achieve a solution of optimal viscosity for controlled delivery and adhesion to the colonic mucosa.

The aqueous diluents may include adjuvants, such as dimethyl sulfoxide (DMSO) up to 2% by weight, and/or glycofurol up to 1% by weight, to promote the uptake of the active ingredients by the tumor. Other adjuvants serving a similar function may also be used together with or in substitution of DMSO or glycofurol; these may be selected from a list comprising polyoxyethylene lauryl ether, sodium taurocholate, and sodium caprate. These adjuvants will be present at concentrations inferior to 1% by weight.

In one embodiment, a dried preparation of a composition according to the present invention may be pre-packaged, for example in single dose units, to be dissolved immediately before administration in the aqueous diluent, which is also supplied pre-packaged in single dose vials.

### Administration

The compositions of the present invention may be applied by administration methods conventionally used in the art for local administration in the lumen of the bowel in the region of a mucosal CRC tumor, adenoma or region of mucosal dysplasia, or in the region of bacterial biofilm located in the colon or rectum.

If the colon and rectum have been cleansed of fecal material by means of a standard regimen of bowel preparation for colonoscopy or surgery, such as the drinking or oral administration of large volumes of liquid containing an appropriate concentration of polyethylene glycol, such as macrogol 3350 or 4000, with or without electrolytes, and the tumor or biofilm is located in the rectum or lower part of the sigmoid colon, a solution of the composition is given as a retention enema into the bowel lumen at the level of the tumor. In certain circumstances, the composition may be given in a larger volume of enema for the treatment of pathology in more proximal parts of the colon.

For tumors and biofilm located in more proximal parts of the colon, the solution of the composition can be administered at the level of the pathology during colonoscopy.

If, in exceptional circumstances, a rectal or sigmoid tumor is to be treated when no bowel preparation has been carried out, the administration of a solution of the composition can be effected after first emptying the bowel by administering a laxative and then giving a colonic enema to clean the bowel;

For tumors in the rectum and lower sigmoid colon, the volume of the retention enema is 50 mL to100 mL. The patient is instructed to lie down lie down for 5 minutes on each side, back and front, and to retain the enema for at least 15 minutes, if possible. When the tumor is located in more proximal parts of the colon, larger-volume enemas can be given, or the solution of the composition can be instilled into the tumor area during colonoscopy, as stated above.

### Dosage

While it is not intended that the invention should be prescriptive for the clinician's choice of suitable dosages for the individual case, it is suggested that the composition be formulated such that a single unit dose of fosfomycin in the present compositions is in the range of 400 milligram to 4 gram. The single unit dose of metronidazole or a carbapenem when used as an additional antibiotic in these compositions may be in the range of 100 milligram to 1 gram. These doses may be exceeded at the discretion of the physician, for example if a large volume of enema is to be given.

A typical single dose may, by the way of an example, consist of 1 gram of fosfomycin dissolved in 50 mL of aqueous diluent, i.e. constituting an approximately 2% weight/volume solution. If metronidazole or a carbapenem is required as an additional antibiotic, it may be added as, for example, 250 milligram in the same diluent, i.e. constituting an approximately 0.5% weight/volume solution.

The above single dose may be augmented proportionately in dosage and volume, maintaining the same concentrations of fosfomycin and, if present, an above-mentioned additional antibiotic, to a maximum volume of 200 mL of diluent containing 4 gram of fosfomycin, and, if present, 1 gram of said additional antibiotic.

If a larger volume of solution is required for a single dose, this can be prepared by diluting a single dose as described above with isotonic saline, without increasing the amount of fosfomycin to be administered above 4 gram, or the amount of additional antibiotic to be administered above 1 gram. Alternatively, the dosage of the antibiotics can be increased at the discretion of the physician, up to a maximum of 20 g of fosfomycin in one liter in any one day, while ensuring that the solution is isotonic.

A dose can preferably be administered once a day, or if the treating clinician considers it necessary and practicable, twice a day such that a patient receives two typical single doses per day on two separate occasions, or even three times a day such that a patient receives three typical single doses per day on three separate occasions, and exceptionally four times a day such that a patient receives four typical single doses per day on four separate occasions, five times a day or six times a day.

Duration of the above administration regime will typically be short, ranging from 1 day to 2 days, but if the treating clinician considers an extension of treatment necessary to achieve the desired result, the dosage regime can be extended to 14 days, such as in the range of 3 days to 5 days, for example 4 days, or in the range of 5 to 7 days, for example 6 days, or in the range of 7 days to 14 days. Treatment can also be given for 1 or 2 days, followed by a pause and then repeated after one week or a fortnight. Compositions according to the present invention may comprise fosfomycin such that single doses are usually in the range of 400 milligram to 4 gram dissolved in a volume of aqueous diluent in the range of 20 mL to 200 mL.

Preferably, a single dose may contain approximately 2% to 2.5% weight/volume of total active ingredients in a solution. Preferably, a single dose is present in isotonic solution. In some embodiments, the compositions of the invention are for administration once or several times, such as twice, three times or four times, after which the treatment is paused and only reinitiated as the attending physician requires.

### Methods of treatment

The present disclosure presents methods to reduce or eliminate infection or colonization of colorectal cancers by fusobacteria as well as to eliminate colonic bacterial biofilm, using local administration of the compositions of the invention into the bowel.

### References

Bergan T (1990) Degree of absorption, pharmacokinetics of fosfomycin trometamol and duration of urinary antibacterial activity. Infection 18 Suppl 2:S65-S69.
Brown ED, Vivas El, Walsh CT, Kolter R (1995) MurA (MurZ), the enzyme that catalyzes the first committed step in peptidoglycan biosynthesis, is essential in Escherichia coli. J Bacteriol 177:4194-4197.
Castellarin M, Warren RL, Freeman JD, Dreolini L, Krzywinski M, Strauss J, Barnes R, Watson P, Allen-Vercoe E, Moore RA, Holt RA (2012) Fusobacterium nucleatum infection is prevalent in human colorectal carcinoma. Genome Res 22:299-306.
Chen Y, Peng Y, Yu J, Chen T, Wu Y, Shi L, Li Q, Wu J, Fu X (2017). Invasive Fusobacterium nucleatum activates beta-catenin signaling in colorectal cancer via a TLR4/P-PAK1 cascade. Oncotarget 8:31802-31814.
Christensen BG, Leanza WJ, Beattie TR, Patchett AA, Arison BH, Ormond RE, Kuehl FA Jr, Albers-Schonberg G, Jardetzky O (1969) Phosphonomycin: structure and synthesis. Science 166:123-125.
Chung L, Thiele Orberg E, Geis AL, Chan JL, Fu K, DeStefano Shields CE, Dejea CM, Fathi P, Chen J, Finard BB, Tam AJ, McAllister F, Fan H, Wu X, Ganguly S, Lebid A, Metz P, Van Meerbeke SW, Huso DL, Wick EC, Pardoll DM, Wan F, Wu S, Sears CL, Housseau F (2018) Bacteroides fragilis toxin coordinates a pro-carcinogenic inflammatory cascade via targeting of colonic epithelial cells. Cell Host Microbe 23:203-214.
Dejea CM, Sears CL (2016) Do biofilms confer a pro-carcinogenic state? Gut Microbes 7:54-57.
Drewes JL, Housseau F, Sears CL (2016) Sporadic colorectal cancer: microbial contributors to disease prevention, development and therapy. Br J Cancer 115:273-280.
Flanagan L, Schmid J, Ebert M, Soucek P, Kunicka T, Liska V, Bruha J, Neary P, Dezeeuw N, Tommasino M, Jenab M, Prehn JH, Hughes DJ (2014) Fusobacterium nucleatum associates with stages of colorectal neoplasia development, colorectal cancer and disease outcome. Eur J Clin Microbiol Infect Dis 33:1381-1390.
Gobernado M (2003) Fosfomycin. Rev Esp Quimioter 16:15-40.
Hand WL, King-Thompson N, Holman JW (1987) Entry of roxithromycin (RU 965), imipenem, cefotaxime, trimethoprim, and metronidazole into human polymorphonuclear leukocytes. Antimicrob Agents Chemother 31:1553-1557.
Hendlin D, Stapley EO, Jackson M, Wallick H, Miller AK, Wolf FJ, Miller TW, Chaiet L, Kahan FM, Foltz EL, Woodruff HB, Mata JM, Hernandez S, Mochales S (1969) Phosphonomycin, a new antibiotic produced by strains of streptomyces. Science 166:122-123.
Höger PH, Seger RA, Schaad UB, Hitzig WH (1985) Chronic granulomatous disease: uptake and intracellular activity of fosfomycin in granulocytes. Pediatr Res 19:38-44.
Johnson CH, Dejea CM, Edler D, Hoang LT, Santidrian AF, Felding BH, Ivanisevic J, Cho K, Wick EC, Hechenbleikner EM, Uritboonthai W, Goetz L, Casero RA Jr, Pardoll D8, White JR, Patti GJ, Sears CL, Siuzdak G (2015) Metabolism links bacterial biofilms and colon carcinogenesis. Cell Metab 21:891-897.
Karageorgopoulos DE, Wang R, Yu XH, Falagas ME (2012) Fosfomycin: evaluation of the published evidence on the emergence of antimicrobial resistance in Gram-negative pathogens. J Antimicrob Chemother 67:255-268.
Kostic AD, Gevers D, Pedamallu CS, Michaud M, Duke F, Earl AM, Ojesina Al, Jung J, Bass AJ, Tabernero J, Baselga J, Liu C, Shivdasani RA, Ogino S, Birren BW, Huttenhower C, Garrett WS, Meyerson M (2012) Genomic analysis identifies association of Fusobacterium with colorectal carcinoma. Genome Res 22:292-298.
Martinez G, Pérez DS, Soraci AL, Tapia MO (2013) Penetration of fosfomycin into IPEC-J2 cells in the presence or absence of deoxynivalenol. PLoS One 8(9):e75068.
Reffert JL, Smith WJ (2014) Fosfomycin for the treatment of resistant gram-negative bacterial infections. Insights from the Society of Infectious Diseases Pharmacists. Pharmacotherapy 34:845-857.
Rubinstein MR, Wang X, Liu W, Hao Y, Cai G, Han YW (2013) Fusobacterium nucleatum promotes colorectal carcinogenesis by modulating E-cadherin/β-catenin signaling via its FadA adhesin. Cell Host Microbe 14:195-206.
Yamamura K, Baba Y, Miyake K, Nakamura K, Shigaki H, Mima K, Kurashige J, Ishimoto T, Iwatsuki M, Sakamoto Y, Yamashita Y, Yoshida N, Watanabe M, Baba H (2017) Fusobacterium nucleatum in gastroenterological cancer: Evaluation of measurement methods using quantitative polymerase chain reaction and a literature review. Oncol Lett 14:6373-6378.
Yang Y, Weng W, Peng J, Hong L, Yang L, Toiyama Y, Gao R, Liu M, Yin M, Pan C, Li H, Guo B, Zhu Q, Wei Q, Moyer MP, Wang P, Cai S, Goel A, Qin H, Ma Y (2017) Fusobacterium nucleatum increases proliferation of colorectal cancer cells and tumor development in mice by activating Toll-like receptor 4 signaling to nuclear factor-κB, and up-regulating expression of microRNA-21. Gastroenterology 152:851-866.
Yu T, Guo F, Yu Y, Sun T, Ma D, Han J, Qian Y, Kryczek I, Sun D, Nagarsheth N, Chen Y, Chen H, Hong J, Zou W, Fang JY (2017) Fusobacterium nucleatum promotes chemoresistance to colorectal cancer by modulating autophagy. Cell 170:548-563.

## Claims

1. A composition comprising fosfomycin as an active ingredient for use in reducing or eliminating infection or colonization of colorectal cancer tumors by fusobacteria, including *Fusobacterium nucleatum,* and the bacterial biofilm associated with such tumors, wherein the composition is administered locally into the bowel lumen.

2. The composition for use according to claim 1, in which the composition further comprises one or more additional antimicrobial or antibiotic agents as active ingredients.

3. The composition for use according to claim 2 above, wherein the one or more additional antimicrobial or antibiotic agents is/are chosen from a list comprising metronidazole or a carbapenem such as ertapenem, or meropenem, or imipenem.

4. The composition for use according to claims 1 to 3, wherein the active ingredient fosfomycin is in the form of fosfomycin trometamol.

5. The composition for use according to claims 1 to 3, wherein the active ingredient fosfomycin is in the form of fosfomycin disodium.

6. The composition for use according to claims 1 to 5 above, wherein the active ingredients are formulated as a dry powder or granulate to be dissolved in an aqueous medium before delivery into the bowel lumen.

7. The composition for use according to claim 6, wherein said local administration is a retention enema or a larger-volume enema.

8. The composition for use according to claim 6, wherein said local administration is at the level of the tumor or the bacterial biofilm via a colonoscope.

9. A composition for use according to claim 6, wherein the dry powder or granulate, and/or the aqueous medium for dissolution, contains one or more additional substances chosen from the list of dimethyl sulfoxide, glycofurol, polyoxyethylene lauryl ether, sodium taurocholate, and sodium caprate.

10. The composition for use according to any of the preceding claims, wherein the composition is administered during neoadjuvant radiotherapy or chemotherapy, e.g. with bevacizumab and/or cytotoxic drugs.

11. A composition for use according to any one of the preceding claims, wherein the composition is administered in combination with the systemic administration of antibiotics to reduce or eliminate fusobacteria or other live microorganisms from tumor cells or metastases remote from the bowel wall.

## Patentansprüche

1. Zusammensetzung, umfassend Fosfomycin als ein Wirkstoff zur Verwendung beim Reduzieren oder Beseitigen von Infizierung oder Kolonisierung kolorektaler Krebstumoren durch Fusobacteriales, einschließlich *Fusobacterium nucleatum,* und den bakteriellen Biofilm, der mit solchen Tumoren assoziiert wird, wobei die Zusammensetzung lokal in das Darmlumen verabreicht wird.

2. Zusammensetzung zur Verwendung nach Anspruch 1, wobei die Zusammensetzung ferner ein oder mehrere zusätzliche antimikrobielle oder antibiotische Mittel als Wirkstoffe umfasst.

3. Zusammensetzung zur Verwendung nach Anspruch 2, wobei das eine oder die mehreren zusätzlichen antimikrobiellen oder antibiotischen Mittel aus einer Liste ausgewählt wird/werden, die Metronidazol oder ein Carbapenem wie etwa Ertapenem oder Meropenem oder Imipenem umfasst.

4. Zusammensetzung zur Verwendung nach den Ansprüchen 1 bis 3, wobei der Wirkstoff Fosfomycin in der Form von Fosfomycintrometamol ist.

5. Zusammensetzung zur Verwendung nach den Ansprüchen 1 bis 3, wobei der Wirkstoff Fosfomycin in der Form von Fosfomycindisodium ist.

6. Zusammensetzung zur Verwendung nach den Ansprüchen 1 bis 5, wobei die Wirkstoffe als ein Trockenpulver oder Granulat formuliert sind, das vor Bereitstellen in das Darmlumen in einem wässrigen Medium aufzulösen ist.

7. Zusammensetzung zur Verwendung nach Anspruch 6, wobei die lokale Verabreichung ein Retentionsklistier oder ein Klistier größeren Volumens ist.

8. Zusammensetzung zur Verwendung nach Anspruch 6, wobei die lokale Verabreichung auf der Ebene des Tumors oder des bakteriellen Biofilms über ein Koloskop erfolgt.

9. Zusammensetzung zur Verwendung nach Anspruch 6, wobei das Trockenpulver oder Granulat und/oder das wässrige Medium zum Auflösen eine oder mehrere zusätzliche Substanzen beinhaltet, die aus der Liste von Dimethylsulfoxid, Glycofurol, Polyoxyethylen-Lauryl-Ether, Natriumtaurocholat und Natriumcaprat ausgewählt ist/sind.

10. Zusammensetzung zur Verwendung nach einem der vorstehenden Ansprüche, wobei die Zusammensetzung während neoadjuvanter Radiotherapie oder Chemotherapie verabreicht wird, z. B. mit Bevacizumab und/oder zytotoxischen Arzneimitteln.

11. Zusammensetzung zur Verwendung nach einem der vorstehenden Ansprüche, wobei die Zusammensetzung in Verbindung mit der systemischen Verabreichung von Antibiotika verabreicht wird, um Fusobacteriales oder andere lebendige Mikroorganismen aus Tumorzellen oder von der Darmwand entfernten Metastasen zu reduzieren oder zu beseitigen.

## Revendications

1. Composition comprenant de la fosfomycine comme ingrédient actif destinée à être utilisée pour réduire ou éliminer l'infection ou la colonisation de tumeurs du cancer colorectal par des fusobactéries, y compris *Fusobacterium nucleatum,* et le biofilm bactérien associé à de telles tumeurs, dans laquelle la composition est administrée localement dans la lumière intestinale.

2. Composition destinée à être utilisée selon la revendication 1, dans laquelle la composition comprend en outre un ou plusieurs agents antimicrobiens ou antibiotiques supplémentaires comme ingrédients actifs.

3. Composition destinée à être utilisée selon la revendication 2 ci-dessus, dans laquelle les un ou plusieurs agents antimicrobiens ou antibiotiques supplémentaires sont choisis dans une liste comprenant le métronidazole ou un carbapénème tel que l'ertapénem, ou le méropénem, ou l'imipénem.

4. Composition destinée à être utilisée selon les revendications 1 à 3, dans laquelle l'ingrédient actif fosfomycine est sous la forme de fosfomycine trométamol.

5. Composition destinée à être utilisée selon les revendications 1 à 3, dans laquelle l'ingrédient actif fosfomycine est sous la forme de fosfomycine disodique.

6. Composition destinée à être utilisée selon les revendications 1 à 5 ci-dessus, dans laquelle les ingrédients actifs sont formulés sous forme de poudre sèche ou de granulats à dissoudre dans un milieu aqueux avant leur administration dans la lumière intestinale.

7. Composition destinée à être utilisée selon la revendication 6, dans laquelle ladite administration locale est un lavement de rétention ou un lavement de plus grand volume.

8. Composition destinée à être utilisée selon la revendication 6, dans laquelle ladite administration locale se fait au niveau de la tumeur ou du biofilm bactérien via un coloscope.

9. Composition destinée à être utilisée selon la revendication 6, dans laquelle la poudre sèche ou le granulat, et/ou le milieu aqueux pour dissolution, contient une ou plusieurs substances supplémentaires choisies dans la liste du diméthylsulfoxyde, du glycofurol, du lauryléther de polyoxyéthylène, du taurocholate de sodium et du caprate de sodium.

10. Composition destinée à être utilisée selon l'une quelconque des revendications précédentes, dans laquelle la composition est administrée pendant une radiothérapie ou une chimiothérapie néoadjuvante, par exemple avec du bévacizumab et/ou des médicaments cytotoxiques.

11. Composition destinée à être utilisée selon l'une quelconque des revendications précédentes, dans laquelle la composition est administrée en combinaison avec l'administration systémique d'antibiotiques pour réduire ou éliminer les fusobactéries ou d'autres micro-organismes vivants des cellules tumorales ou des métastases éloignées de la paroi intestinale.
